## Europäisches Patentamt
## European Patent Office
## Office européen des brevets

(19)

(11) Numéro de publication: **0 183 758**
**B1**

(12) ## FASCICULE DE BREVET EUROPEEN

(45) Date de publication du fascicule du brevet: **07.11.90**

(51) Int. Cl.⁵: **A 61 L 31/00,** A 61 L 27/00

(21) Numéro de dépôt: **85902583.5**

(22) Date de dépôt: **17.06.85**

(86) Numéro de dépôt international:
**PCT/FR85/00154**

(87) Numéro de publication internationale:
**WO 86/00230 16.01.86 Gazette 86/02**

(54) **PIECE CHIRURGICALEMENT IMPLANTABLE.**

(30) Priorité: **19.06.84 FR 8409573**

(43) Date de publication de la demande:
**11.06.86 Bulletin 86/24**

(45) Mention de la délivrance du brevet:
**07.11.90 Bulletin 90/45**

(84) Etats contractants désignés:
**BE CH DE GB IT LI**

(56) Documents cités:
**WO-A-81/00808**
**FR-A-2 105 998**
**FR-A-2 135 326**
**FR-A-2 350 825**
**FR-A-2 427 197**
**FR-A-2 427 315**
**GB-A-2 092 891**

(73) Titulaire: **AEROSPATIALE Société Nationale Industrielle**
**37, Boulevard de Montmorency**
**F-75781 Paris Cédex 16 (FR)**

(72) Inventeur: **RENARD, Pierre**
**3, Allée des Glands**
**F-91760 Itteville (FR)**
Inventeur: **CHAREIRE, Jean-Louis**
**66, rue Aristide Briand**
**F-92300, Levallois Perret (FR)**

(74) Mandataire: **Bonnetat, Christian et al**
**CABINET BONNETAT 23, Rue de Léningrad**
**F-75008 Paris (FR)**

Courier Press, Leamington Spa, England.

## Description

La présente invention concerne un procédé pour la réalisation d'une pièce implantable par voie chirurgicale dans un organisme et la pièce ainsi obtenue comme defini dans les revendications

On sait que, si un corps étranger est introduit dans un organisme, un mécanisme de défense naturelle de celui-ci pousse ses cellules à isoler ledit corps étranger. Par exemple, si une écharde est enfoncée dans la peau, les cellules de l'épiderme l'entourent d'une enveloppe étanche et la coutournent afin de tenter de la rejeter à l'extérieur. De plus, si un corps étranger de grande longueur, tel qu'un fil ou un tube, traverse la peau, celle-ci recouvre sur toute son épaisseur ledit corps d'une gaine non adhérente de cellules épithéliales. Dans ce cas, il est évident que des microbes peuvent s'introduire en permanence entre le corps étranger et ladite gaine, de sorte qu'il en résulte un foyer d'infection.

La présente invention a pour objet de remédier à ces inconvénients et de permettre la réalisation d'une pièce biocompatible implantable par voie chirurgicale susceptible non seulement de ne pas être rejetée par ledit organisme, mais encore de se solidariser des tissus dans lesquels elle est implantée.

A cette fin, selon l'invention, le procédé pour la réalisation d'une pièce à base de fibres de carbone implantable par voie chirurgicale dans un organisme et étant destinée à former un dispositif de traversée percurquée, est remarquable en ce que

—on réalise un lit de tronçons de fibres de carbone dont la longueur est au plus égale à 30 mm, lesdits tronçons reposant librement les uns sur les autres avec des orientations aléatoires;

—ou soumet ledit lit à une compression de façon que sa masse spécifique soit comprise entre 0,05 et 0,3 g/cm³;

—on introduit ledit lit ainsi comprimé dans une enceinte enfermant un gaz hydrocarburé et on soumet ledit gaz aux conditions de son cracking, de sorte que du pyrocarbone se dépose sur les tronçons de fibres composant ledit lit; et,

—on poursuite le cracking dudit gaz hydrocarburé jusqu'à ce que ledit lit de tronçons de fibres de carbone atteigne une masse spécifique comprise entre 0,5 et 1,6 g/cm³.

On obtient ainsi une structure composite poreuse biocompatible constituée d'une armature de carbone dont les tronçons de fibres sont enrobés par une matrice de pyrocarbone. Des essais ont montré que les cellules des tissus entourant une pièce implantée ayant une telle structure présentaient une affinité telle avec ladite pièce qu'elles se fixaient solidement à elle avant que le phénomène de rejet puisse commencer à se manifester. Par suite, une pièce implantable selon l'invention, non seulement n'est pas rejetée par l'organisme, mais encore est fermement liée aux tissus vivants qui l'entourent, puisque les cellules desdits tissus (fibroblasts) la colonisent sur toute son épaisseur.

On remarquera que les brevets US—A— 3 992 725 et US—A—4 129 470 décrivent déjà une pièce chirurgicalement implantable à base de fibres de carbone. Cependant, dans ce cas, les fibres de carbone sont liées les unes aux autres par du polytétrafluoroéthylène, de sorte que les pores de la structure soit en grande majorité bouchés. Il en résulte que les emplacements d'ancrage des tissus vivants sur la pièce ne sont pas aussi nombreux qu'ils pourraient l'être et que la rapidité et la qualité de la liaison entre la pièce et les tissus ne sont pas optimales.

Dans la présente invention, au contraire, lors du cracking du gas hydrocarburé, le pyrocarbone se dépose uniquement sur les tronçons de fibres en faisant grossir le diamètre de ceux-ci, et un tel dépôt s'effectue pratiquement de façon uniforme sur toute la longueur desdits tronçons, puisque le gaz hydrocarburé passe librement entre les tronçons de carbone constituant le lit comprimé. Par suite, quoique le dépôt de pyrocarbone réduise les dimensions des pores existant entre les tronçons de fibres du lit comprisé, le nombre desdits pores reste le même, de sorte que la pièce obtenue est particulièrement poreuse et présente des pores communicants.

On comprendra aisément qu'en contrôlant le cracking du gaz hydrocarburé, il est possible d'obtenir le meilleur compromis possible entre les dimensions des pores de la pièce et la solidité de celle-ci, donnée par la matrice de pyrocarbone.

Lorsque l'on désire conférer à la pièce obtenue par la mise en oeuvre du procédé selon l'invention, des propriétés mécaniques renforcées dans une direction perpendiculaire audit lit, par exemple pour prévoir des surfaces d'usure ou des trous orientés de cette manière, il est avantageux de soumettre ledit lit de tronçons de fibres de carbone, de préférence après compression, à un aiguilletage de direction orthogonale aux multiples directions coplanaires desdits tronçons. On obtient ainsi une sorte de feutre aiguilleté de fibres de carbone, qui, ensuite, est éventuellement comprimé et reçoit la matrice de pyrocarbone.

Pour donner sa forme définitive à la pièce obtenue par la mise en oeuvre du procédé selon l'invention, il peut être nécessaire de l'usiner. Dans ce cas, afin d'éviter, d'une part, que des copeaux d'usinage puissent rester fixés à la pièce usinée et se répandre par la suite dans l'organisme et, d'autre part, que des particules superficielles fragiles se détachent de la pièce après implantation, on soumet ladite pièce usinée à un nettoyage très actif que comporte non seulement un dépoussiérage soigné, mais encore l'application d'ultrasons pour éliminer lesdites particules faiblement solidaires du reste de ladite pièce.

Un tel nettoyage peut également comporter une oxydation de la pièce usinée à température élevée (par exemple 1000°C), pendant quelques secondes sous oxygène. Une telle oxydation permet, non seulement la combustion et l'élimination de particules libres provenant d'usinage ainsi que l'arrondissement des micro-aspérités fragiles, mais encore la formation de groupements oxygénés, par exemple du type carboxyle, qui se fixent sur la pièce. Ultérieurement, si l'on soumet ladite pièce à un chauffage (par exemple 1500°C) sous vide ($10^{-3}$ torrs), l'oxygène de ces groupements oxygénés se dégage, de sorte qu'il apparaît des radicaux libres, permettant le greffage d'atomes ou de molécules (lactide, muco-polysaccharide, halogène, ...) susceptible de favoriser l'accrochage des cellules vivantes. dans ce cas, la fixation des cellules vivantes sur la pièce n'est dont pas seulement physique (accrochage des cellules dans les pores de la pièce), mais encore chimique.

Il est de plus avantageux, avant implantation de la pièce dans l'organisme, de l'imprégner d'un sérum nutritif pour les cellules. Ainsi, on favorise la vie des cellules pendant leur croissance initiale à l'intérieur des pores de la pièce. Un tel sérum nutritif peut être du type de celui utilisé pour les cultures *in vitra* des cellules analogues.

Ainsi, une pièce implantable par voie chirurgicale dans un organisme est remarquable en ce qu'elle présente une structure composite comportant une armature de tronçons de fibres de carbone dont la longueur est au plus égale à 30 mm et qui forment un lit dans lequel lesdits tronçons sont enrobés de pyrocarbone les liant les uns aux autres, la masse spécifique de cette structure composite étant comprise entre 0,5 et 1,6 g/cm.

De préférence, ladite armature forme un feutre aiguilleté de tronçons de fibres de carbone et le diamère desdites fibres est de l'ordre de 8 à 12 microns.

Quoique non exclusivement, la pièce implantable selon l'invention est particulièrement adaptée à la réalisation d'un dispositif de traversée percutanée, tel que par exemple décrit dans les brevets US—A—3 663 965, US—A—3 783 868, US—A—4 321 914, US—A—4 344 435 et GB—A—2 056 282, comprenant une partie tubulaire solidaire d'une collerette à une de ses extrémités.

De préférence, selon une particularité de l'invention, la paroi extérieure de la partie tubulaire présente la forme d'un cône coaxial à ladite partie tubulaire et se raccordant à ladite collerette, du côté de sa base. L'inclinaison des génératrices du cône par rapport à ladite collerette est avantageusement compris entre 30 et 60°.

Afin d'augmenter encore la solidité de la liaison entre la pièce et les tissus vivants, au moins une rainure annulaire, à bords arrondis et concentrique à ladite partie tubulaire, est prévue dans la face de la collerette dirigée vers celle-ci. Ainsi, les cellules desdits tissus vivants peuvent peupler ladite rainure et s'y accrocher.

Une telle rainure peut avoir une profondeur et une largeur de l'order du mm.

Pour maintenir fermement en ce place ladite pièce implantable, avant et pendant le développement des cellules dans ladite pièce, il est avantageux que celle-ci comporte un chapeau amovible, disposé à l'extérieur de la peau et pourvu de pointes dirigées vers celle-ci, ainsi que des moyens de serrage pour presser les pointes dudit chapeau contre la peau. Après cicatrisation complète, un tel chapeau peut être éliminé en remplacé par un bouchon ou analogue.

Les figures du dessin annexé feront bien comprendre comment l'invention peut être réalisée. Sur ces figures, des références identiques désignent des éléments semblables.

La figure 1 montre, en coupe axiale schématique, un dispositif de traversée percutanée selon l'invention.

La figure 2 montre, en vue semblable à la figure 1, une variante de réalisation du dispositif de traversée percutanée selon l'invention.

La figure 3 illustre, en coupe axiale, une autre variante de réalisation de la pièce composite de carbone du dispositif de traversée percutanée selon l'invention.

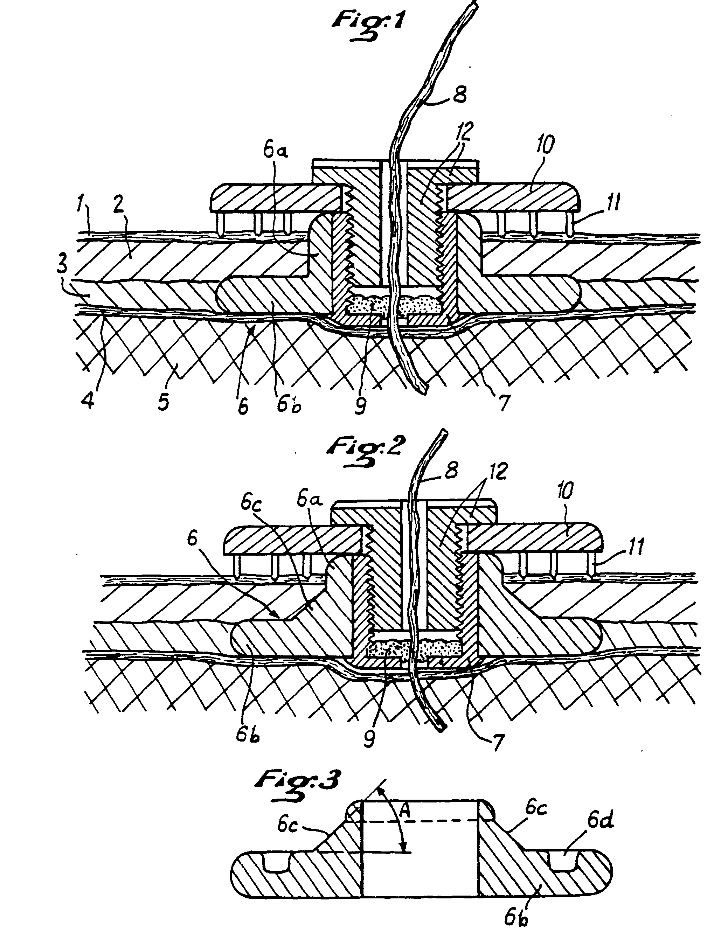

Comme le montrent ls figures 1 et 2, la peau, à travers laquelle le mode de réalisation décrit du dispositif selon l'invention doit permettre un passage, comporte plusieurs couches superposées qui, de l'extérieur vers l'intérieur sont lépiderme 1, le derme 2, une première couche de tissu adipeux 3, le fascia superficiel 4 et une seconde couche de tissu adipeux 5.

Le dispositif de traversée percutanée selon l'invention comporte une pièce implantable 6, constituée d'une partie tubulaire 6a solidaire d'une collerette 6b à une de ses extrémités. L'épaisseur de la collerette 6b peut être de l'ordre de 2 mm.

Par voir chirurgicale, la pièce implantable 6 est mise en place de façon que sa collerette 6b soit situé juste sous le derme 2, à l'intérieur de la première couche de tissu adipeux 3 et au-dessus du fascia superficiel 4, et que sa partie tubulaire 6a traverse l'épiderme 1 et le derme 2.

A l'intérieur de la partie tubulaire 6a de la pièce implantable 6 est disposée une chemise 7, en titane ou en acier inoxydable par exemple, ladite chemise 7 étant solidarisée de la pièce 6, par exemple par collage. Le fond de la chemise 7 est percé pour permettre le passage d'un élément 8, tel qu'un câble, tuyau, etc ... reliant l'intérieur d'un patient à l'extérieur.

Une masse d'étanchéité 9, par exemple une résine époxy à durcissement rapide, assure l'étanchéité entre l'élément 8 et la chemise 7.

Pour assurer le bon maintien en position de la peau après implantation et pendant toute la durée de la cicatrisation, on prévoit une rondelle 10, pourvue d'ergots pointus 11 sur sa face destinée à être dirigée vers l'épiderme 1. Ces ergots 11, par exemple, sont répartis tous les 2 ou 3 mm et présentent un diamètre de 0,2 mm. La rondelle 10 est maintenue par une vis 12 coopérant avec un filetage intérieur de la chemise 7.

Les ergots 11 pénètrent dans l'épiderme 1, ce qui permet de maintenir efficacement la peau en place et de la plaquer contre la collerette 6b. De plus, l'espace ménagé entre la rondelle 11 et l'épiderme 1 favorise d'aération de la plaie et dont la cicatrisation.

Après cicatrisation définitive, la rondelle 10 et la vis 12 sont remplacées par un bouchon protecteur (non représenté), vissé dans la chemise 7 et prenant appui sur l'extrêmité libre de la partie tubulaire 6a de la pièce 6. Un tel bouchon est facilement démontable pour permettre les vérifications nécessaires.

Sur la figure 1, la paroi extérieure de la partie tubulaire 6a est cylindrique. En revanche, dans les variantes de réalisation des figures 2 et 3, la paroi extérieure 6c de la partie tubulaire 6a est conique, la base de cette partie conique se raccordant à la collerette 6b. Un tel mode de réalisation est préférable pour éviter d'éventuelles poches dans lesquelles les cellules épithéliales pourraient former une gaine de rejet de la pièce implantable 6. L'angle A du cône peut être compris entre 30 et 60°.

De plus, comme le montre la figure 3, on peut prévoir une cavité annulaire 6d dans la face de la collerette 6b dirigée vers la partie tubulaire 6a. Une telle cavité annulaire 6d, de préférence à bords arrondis, peut avoir une profondeur et une largeur de l'ordre de 1 mm. Elle permet d'augmenter la résistance au déchirement, dès qu'elle est peuplée par les cellules du derme. Son bord intérieur peut être situé à environ 1 mm de la base du cône 6c.

Conformément à l'invention, pour obtenir la pièce implantable 6 des figures 1 à 3, on commence à réaliser un lit de tronçons de fibres de carbone d'environ 8 microns de diamètre et de quelques mm de longeuur, par exemple en versant ces tronçons de fibres de carbone sur un support horizontal. Dans ce lit, les tronçons de fibre de carbone occupent des positions plus ou moins horizontales de direction aléatoire et reposent librement les uns sur les autres. Ensuite, on soumet ledit lit à une compression et à un aiguilletage vertical, de sorte que l'on obtient un feutre de tronçons de fibres de carbone. Ce feutre est alors éventuellement soumis à une compression verticale additionnelle pour ajuster sa masse spécifie à 0,25 g/cm³.

On introduit le feutre comprimé dans une enceinte enfermant du gaz de ville sous une pression de 15 torrs et on procède au cracking dudit gaz à 1000°C. On arrête le cracking du gaz lorsque la masse spécifique du feutre atteint 1 g/cm³.

On obtient alors une matière très poreuse, à pores ouverts. Ces pores ont des dimensions transversales extrêmes d'environ 0,2 mm à 0,005 mm.

Dans cette matière ainsi obtenue, on usine la pièce 6 de façon que la partie tubulaire 6a soit parallèle aux fibres aiguilletées verticalement et que la collerette 6b soit parallèle aux fibres horizontales initiales.

La pièce 6 usinée est soumise à dépoussièrage, à l'action d'un générateur d'ultra-sons et à une oxydation à température élevée et pendant quelques secondes.

Après un éventuel greffage d'atomes ou de molécules favorisant la vitesse d'accrochage et/ou l'adhésion des cellules du derme (comme indiqué ci-dessus) et imprégnation de sérum nutritif, la pièce 6 est mise en place de la façon décrite ci-dessus.

Au course de la cicatrisation, la pièce implantable 6 sera rendue solidaire de la peau, principalement du fait de la pénétration des fibroblasts du derme dans toute l'épaisseur de la collerette 6b.

## Revendications

1. Procédé pour la réalisation d'une pièce à base de fibres de carbone revêtues de pyrocarbone, ladite pièce étant implantable par voie chirurgicale dans un organisme et étant destinée à former un dispositif de traversée percutanée, caractérisé en ce que:

—on réalise un lit de tronçons de fibres de carbone dont la longueur est au plus égale à 30 mm, lesdits tronçons reposant librement les uns sur les autres avec des orientations aléatoires;

—on soumet ledit lit à une compression de façon que sa masse spécifique soit comprise entre 0,05 et 0,3 g/cm³;

—on introduit ledit lit ainsi comprimé dans une enceinte enfermant un gaz hydrocarburé et on soumet ledit gaz aux conditions de son cracking, de sorte que du pyrocarbone se dépose sur les tronçons de fibres composant ledit lit; et,

—on poursuite le cracking dudit gaz hydrocarburé jusqu'à ce que ledit lit de tronçons de fibres de carbone atteigne une masse spécifique comprise entre 0,5 et 1,6 g/cm³.

2. Procédé selon la revendication 1, caractérisé en ce que on soumet ledit lit de tronçons de fibres de carbone à un aiguilletage de direction orthogonale aux multiples directions coplanaires desdits tronçons.

3. Procédé selon l'une des revendications 1 ou 2, selon lequel on soumet ladite pièce à un usinage, caractérisé en ce que, après usinage, on soumet ladite pièce à un dépoussiérage et à l'action d'ultra-sons.

4. Procédé selon l'une des revendications 1 à 3, caractérisé en ce que, après usinage, on soumet ladite pièce à une oxydation à températue élevée sous oxygène.

5. Procédé selon la revendication 4, caractérisé en ce que les groupements oxygénés résultant de ladite oxydation sont substitués par des atomes ou molécules susceptibles de favoriser l'adhésion chimique et le peuplement de la pièce par les cellules environnantes.

6. Procédé selon l'une des revendications 1 à 5, caractérisé en ce que ladite pièce implantable est imprégnée de sérum nutritif, avant implantation.

7. Pièce à base de fibres de carbone, implantable par voie chirurgicale dans un organisme et destinée à former un dispositif de traversée percutanée, réalisée par la mise en oeuvre du procédé selon l'une quelconque des revendications 1 à 6, et comprenant une partie tubulaire (6a) solidaire d'une collerette (6b) à une de ses extrémités, caractérisée en ce que la paroi extérieure (6c) de la partie tubulaire présente la forme d'un cône coaxial à ladite partie tubulaire et se raccordant à ladite collerette (6b), du côté de sa base.

8. Pièce selon la revendication 7, caractérisée en ce que l'inclinaison des génératrices du cône par rapport à la collerette est compris entre 30° et 60°.

9. Pièce selon l'une des revendications 7 ou 8, caractérisée en ce que au moins une rainure annulaire (6d), à bords arrondis, et concentrique à ladite partie tubulaire, est prévue dans la face de la collerette (6b) dirigée vers celle-ci.

10. Pièce selon l'une des revendications 7 à 9, caractérisée en ce qu'elle comporte un chapeau amovible (10), disposé à l'extérieur de la peau et pourvu de pointes (11) dirigées vers celle-ci, ainsi que des moyens de serrage pour presser les pointes dudit chapeau contre la peau.

**Patentansprüche**

1. Verfahren zum Herstellen eines chirurgischen Implantats auf der Basis von mit Pyrokohlenstoff überzogenen Kohlenstoff-Fasern zur Bildung einer perkutanen Durchführungsvorrichtung in einem Organismus, dadurch gekennzeichnet, daß
—ein Bett aus Kohlenstoff-Faserteilstücke geschaffen wird, dessen Länge höchstens gleich 30 mm ist, wobei die Teilstücke wahlfrei ausgerichtet frei aufeinander ruhen,
—das Bett einem Verdichtungsdruck ausgesetzt wird, so daß seine spezifische Masse zwischen 0,05 und 0,3 g/cm$^3$ liegt,
—das derart komprimierte Bett in eine Einfassung eingeführt wird, die ein zu Kohlenwasserstoff verbundenes Gas einschließt, und das Gas den Krackbedingungen derart ausgesetzt wird, daß sich auch den das Bett bildenden Faserteilstücken Pyrokohlenstoff ablagert, und daß
—das Kracken des zu Kohlenwasserstoff verbundenen Gases so lange fortgeführt wird, bis das Bett aus den Kohlenstoff-Faserteilstücken eine zwischen 0,5 und 1,6 g/cm$^3$ liegende spezifische Masse erreicht.

2. Verfahren nach Anspruch 1, dadurch gekennzeichnet, daß das Bett aus Kohlenstoff-Faserteilstücken einer Nadelbehandlung in einer zu den koplanaren Mehrfachrichtungen der Teilstücken orthogonalen Richtung unterzogen wird.

3. Verfahren nach einem der vorhergehenden Ansprüche 1 oder 2, nach dem das Stück maschinenbearbeitet wird, dadurch gekennzeichnet, daß das Stück nach der Maschinenbearbeitung einer Entstaubungs- und Ultraschallbehandlung ausgesetzt wird.

4. Verfahren nach einem der vorhergehenden Ansprüche 1 bis 3, dadurch gekennzeichnet, daß das Stück nach der Maschinenbearbeitung einer Oxidationsbehandlung unter Sauerstoff bei erhöhter Temperatur unterzogen wird.

5. Verfahren nach Anspruch 4, dadurch gekennzeichnet, daß die mit Sauerstoff verbundenen Zusammenstellungen, die sich aus der Oxidationsbehandlung ergeben, durch Atome oder Moleküle substituiert werden, durch die chemische Adhäsion und die Besetzung des Stückes von umgebenden Zellen begünstigt werden kann.

6. Verfahren nach einem der vorhergehenden Ansprüche 1 bis 5, dadurch gekennzeichnet, daß das implantierfähige Stück vor der Implantation mit einem Nährseum imprägniert wird.

7. Chirurgische Implantat auf Kohlenstoff-Faserbasis, das auf chirurgischem Wege in einen Organismus eingepflanzt werden kann und dazu dient, eine perkutane Durchführungsvorrichtung zu bilden, und das gemäß der Ausführung des Verfahrens nach den Ansprüchen 1 bis 6 geschaffen wird sowie einen röhrenförmigen Teil (6a) aufweist, der mit einem Kragen (6b) an einem seiner Enden verbunden ist, dadurch gekennzeichnet, daß die Außenwand (6c) des röhrenförmigen Teiles die Form eines koaxialen Konus am röhrenförmigen Teil bietet und sich auf der Seite seiner Grundfläche an den Kragen (6b) anschließt.

8. Chirurgische Implantat nach Anspruch 7, dadurch gekennzeichnet, daß die Neigung der Erzeugenden des Konus gegenüber dem Kragen im Bereich von 30° bis 60° liegt.

9. Chirurgisches Implantat nach einem der vorhergehenden Ansprüche 7 oder 8, dadurch gekennzeichnet, daß zumindest eins rundkantige, mit dem röhrenförmigen Teil konzentrische Ringnut (6d) auf der diesem zugewandten Fläche des Kragens 6b) vorgesehen ist.

10. Chirurgisches Implantat nach einem der vorhergehenden Ansprüche 7 bis 9, dadurch gekennzeichnet, daß es eine abnehmbare Abdeckung (10), die außerhalb der Haut angeordnet und mit zu ihr hinweisenden Spitzen (11) versehen ist, sowie Festspannmittel aufweist, um die Stifte der Abdeckung gegen die Haut zu drücken.

**Claims**

1. Process for forming a carbon fiber based piece coated with pyrocarbon, implantable by surgery in an organism and being intended to form a percutaneous passage device, characterized in that:
—a bed is formed of carbon fiber sections whose length is at most equal to 30 mm, said sections freely resting on each other with random orientations;
—said bed is subjected to compression so that its specific mass is between 0.05 and 0.3 g/cm$^3$;
—said bed thus compressed is introduced into an enclosure containing hydrocarbonated gas and said gas is subjected to the conditions of its cracking so that pyrocarbon is deposited on the fiber sections forming said bed; and
—cracking of said hydrocarbonated gas is continued until said bed of carbon fiber sections reaches a specific mass between 0.5 and 1.6 g/cm$^3$.

2. Process according to claim 1, characterized in that said bed of carbon fiber sections is subjected to tufting in a direction orthogonal to the multiple coplanar directions of said sections.

3. Process according to one of claims 1 to 2, in which said piece is subjected to machining, characterized in that, after machining, said piece is subjected to dust removal and to the action of ultrasounds.

4. Process according to one of claims 1 to 3, characterized in that, after machining, said piece is subjected to oxidization at a high temperature in an oxygen atmosphere.

5. Process according to claim 4, characterized in that the oxygenated groups resulting from said oxidization are substituted by atoms or molecules capable of promoting the chemical adhesion and the stocking of the piece by the surrounding cells.

6. Process according to one of claims 1 to 5, characterized in that said implantable piece is impregnated with nutritive serum, before implantation.

7. A carbon fiber based piece, implantable by surgery in an organism and intended to form a percutaneous passage device, made by carrying out the process according to any one of claims 1 to 6, and comprising a tubular part (6a) integral with a collar (6b) at one of its ends, characterized in that the outer wall (6c) of the tubular part has the shape of a cone coaxial with said tubular part and joined to said collar (6b) on the side of its base.

8. Piece according to claim 7, characterized in that the slant of the generatrices of the cone with respect to the collar is between 30 and 60°.

9. Piece according to one of claims 7 or 8, characterized in that at least one annular groove (6d) with rounded edges and concentric to said tubular part is provided in the face of the collar (6b) directed theretowards.

10. Piece according to one of claims 7 to 9, characterized in that it comprises a removable cap (10) disposed outside the skin and having points (11) directed theretowards, as well as clamping means for pressing the points of said cap against the skin.

Fig.1

Fig.2

Fig.3